# EUROPEAN PATENT SPECIFICATION

(11) **EP 0 568 207 B1**
(45) Date of publication and mention of the grant of the patent: **03.03.1999**
(21) Application number: 93302691.6
(22) Date of filing: 06.04.1993
(51) Int. Cl.: A61M 5/32

(54) **Medical device package**
Verpackung für eine medizinische Vorrichtung
Emballage pour dispositif médical

(30) Priority: 27.04.1992 US 874470
(43) Date of publication of application: 03.11.1993
(73) Proprietor: Becton, Dickinson and Company, Franklin Lakes, New Jersey 07417-1880 (US)
(72) Inventor: Brichacek, Robert J., Columbus, Nebraska 68601 (US)
(74) Representative: Ruffles, Graham Keith

(56) References cited:
- EP-A- 0 284 183
- US-A- 4 113 090
- US-A- 4 900 309
- US-A- 4 927 019
- US-A- 4 986 818

## Description

### 1. Background of the Invention

This invention relates to a disposable medical device and its storage in a package which provides a sterility barrier and protection, both for the device and for medical personnel, from any sharp point which may be present on the device.

### 2. Description of Related Information

Generally speaking, packages for medical devices, particularly devices having a sharp point intended to be used to perform percutaneous punctures, have multiple requirements. The package must provide physical protection and function as a sterility barrier for the devices so that the device is presented to its intended user in an undamaged and sterile state. The package should also provide the user easy access to the device inside. Further, it should be easily possible to remove a sterile device from the protective package without contacting the non-sterile outside of the package.

In addition to the attributes related above for the package prior to and during the use of the device contained therein, there exists a need for the package to be able to safely contain the device after its use in a patient, even if the medical operator improperly performs the usage and disposal. There has always been a need to protect medical personnel from inadvertent sticks by used sharp medical devices ("sharps"), but the advent of Human Immunodeficiency Virus (HIV) and the continuing threat of other infectious diseases such as hepatitis have greatly increased awareness and demand for additional protection. Additionally, there exists a need to protect service personnel from medical devices improperly used and disposed of by medical personnel.

U.S. Patent 4,113,090 to Carstens discloses a package for a medical device, particularly a needle assembly. The package includes a hollow shield having an open end for insertion and removal of a medical instrument and means for supporting the instrument inside. This package comprises a shield, a cap and a closure. The cap is shiftable between a first position to cooperate with the shield in protecting the instrument and a second position exposing a portion of the instrument to facilitate grasping and removal. Further, the cap is removable from the shield, thus allowing separation of the cap from the shield. This facile separation renders the prior art device of little value in the now very important area of protection of medical personnel during improper reshielding, which is still widely practiced contrary to recommended practice and manufacturers instructions. Further, if the tube is separated from the shield, service personnel unknowingly handling the improperly reshielded device during disposal subsequent to use will not enjoy the protection afforded by the presence of the secondary barrier.

Although the teachings of Carstens provide some of the attributes of the instant device, the problem of separation of the cap from the shield with the concomitant loss of protection for service personnel continues to provide an opportunity for improvement in this area.

EP 284 183 describes a safety device for a hypodermic needle which device includes a hollow cylindrical portion, having an annular groove therein, disposed around the neck of a hypodermic syringe; a truncated conical surface disposed on the shoulder of the syringe; and a flexible projection abutting the neck. When a sheathed hypodermic needle engages the syringe, the safety device, via the groove therein, engages the sheath and is subsequently removed therewith. Upon recapping the needle, the conical surface shields the user from accidental puncture. Upon disengaging the sheathed needle from the syringe, the flexible projection closes behind the needle, retaining it in the sheath. Other embodiments of the invention include shields for precluding accidental needle puncture, and flexible projections for securing the needle in the sheath.

### Summary of the Invention

A packaged syringe assembly of the present invention comprises:
a syringe having a distal luer tip for detachably mounting a needle and having an external collar on said distal tip;
a needle assembly comprising a proximal luer hub and a needle affixed thereto, said hub detachably mounted on said distal syringe tip;
a needle shield having a distal and a proximal end, a cylindrical sidewall having an external surface sized to removably fit at said proximal end with an interference over at least a portion of said proximal luer hub to enclose the needle of said needle assembly, said shield having a rim extending outwardly from said external surface at said proximal end thereof;
a formed cylindrical hollow tube having a proximal end and a distal end, and external side and an internal side forming a sidewall, said proximal end and said distal end being open so as to form an open bore extending therethrough, said proximal end of said tube having a flange extending outwardly from said external surface thereof, said flange including a planar surface having an opening to said bore said bore having a distal portion and a proximal portion adjacent to said distal portion at an interface, said bore sized so that said distal end of said needle shield fits through said proximal portion and slidably through said distal portion of said bore so that said rim of said shield engages said interface; and
said hollow tube distal portion has a length covering at least a portion of said needle shield, said distal portion having within said bore a plurality of sealing rings sized to fit cooperatively with an interference to said external surface of said needle shield, said cooperation of said rings and said surface functioning as a barrier to passage of microorganisms and said coverage of said needle shield by said tube thereby serving to prevent accidental personnel sticks by serving as a barrier to penetration of the needle through said sidewall of said shield.

The packaged syringe assembly is characterized in that said proximal portion of said bore has an inwardly extending projection located proximally to said interface, said projection being shaped to allow said rim of said shield to be passed thereover with movement in a distal direction and to resist passage of said rim with movement in a proximal direction;
and said proximal portion of said bore has a plurality of raised seal rings located proximally to said inward projection sized to fit cooperatively with said external collar of said syringe luer tip with an interference, said cooperation of said rings and said collar functioning as a barrier to passage of microorganisms.

The presence of the inward projection makes the needle shield difficult to remove unintentionally from the hollow tube. The presence of the hollow tube on the shield provides a secondary barrier to needle sticks for service personnel if a needle should penetrate through the shield when unknowingly handling an improperly reshielded product improperly disposed of.

### Brief Description of the Drawings

- Fig. 1: is a perspective view of the preferred embodiment of the packaging assembly for a needle assembly.
- Fig. 2: is a perspective view of a syringe assembly shown in conjunction with the packaging of a preferred embodiment of the present invention.
- Fig. 3: is a partial cross-sectional view of the packaging assembly of Fig. 1 taken along line 3-3.
- Fig. 4: is a partial cross-sectional view of the syringe assembly of Fig. 2 taken along line 4-4.
- Fig. 5: is, similar to Fig. 3, a partial cross-sectional view of an alternative embodiment of the packaging assembly of Fig. 1.
- Fig. 6: is a partial cross-sectional view of the packaging assembly of Fig. 5 with the microbial barrier opened.

### Detailed Description

For the purposes of the description of the present invention the term "distal end" is meant to refer to the end of the assembly furthest from the operator, whereas the term "proximal end" is meant to refer to the end of the assembly closest to the operator.

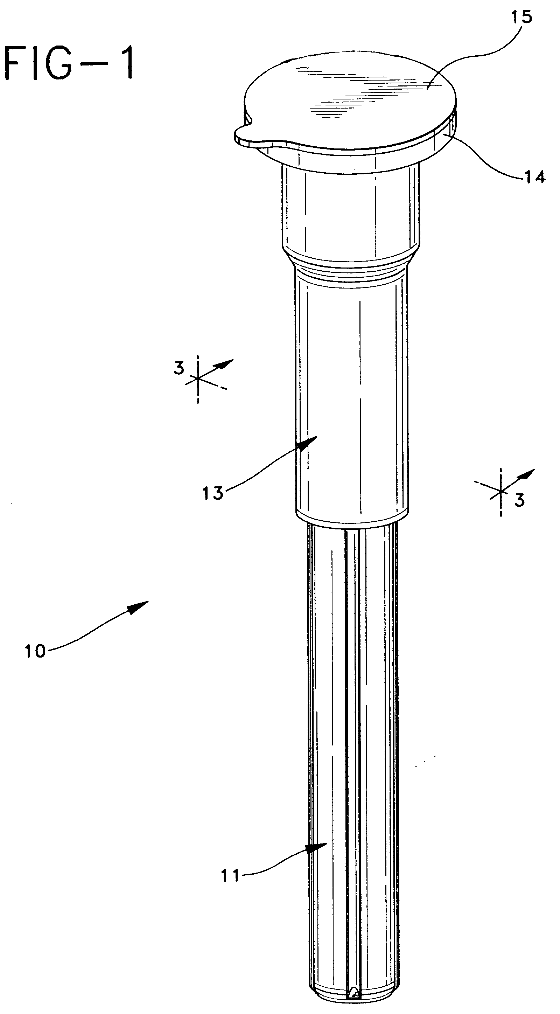

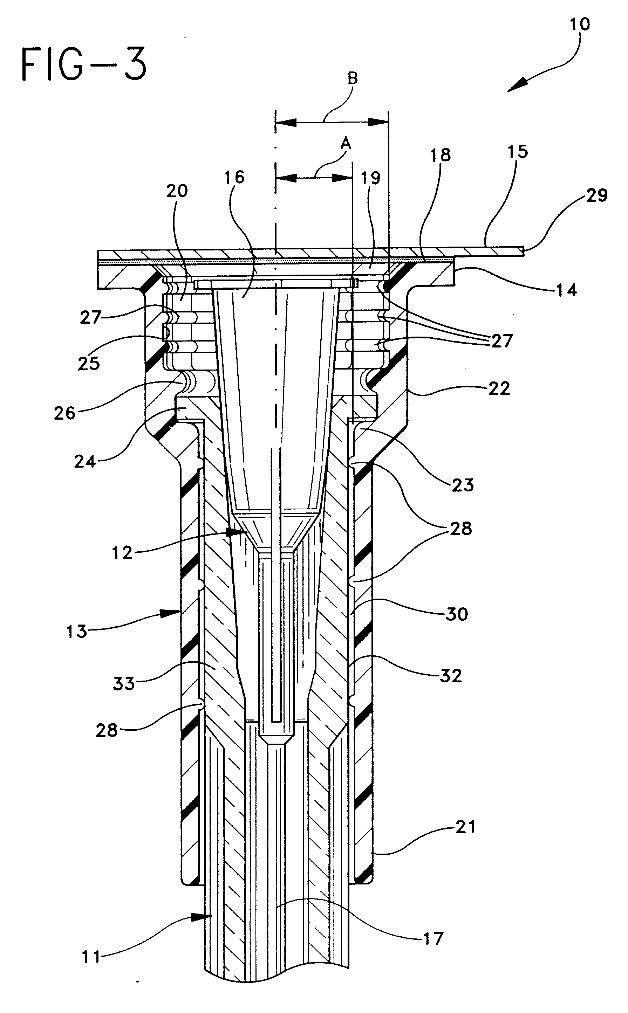

Referring to Figs. 1 and 3, a preferred packaging assembly 10 of the present invention comprises a container, such as a needle shield 11, containing a needle assembly 12, and a generally cylindrical hollow tube 13. This tube includes a proximal flange 14 optionally having a peelable microbial barrier 15 adhesively bonded to the flange. Needle assembly 12 includes a luer needle hub 16 with a needle 17 affixed thereto.

As shown in Fig. 3, proximal flange 14 has a generally planar surface 18 having an opening 19 to a tube bore 20. Tube 13 has a distal portion 21 with a radius A and a proximal portion 22 with a radius B adjacent at an interface 23. Radius B is larger than radius A. Needle shield 11 has an outwardly extending rim 24 that fits within proximal portion 22 of bore 20 and distal portion 21 is sized so that shield 11 fits slidably within it up to rim 24. Rim 24 passes through proximal portion 22 to rest on interface 23 between portions 21 and 22.

An inner wall 25 of proximal portion 22 has an inward projection 26 located proximally from interface 23. The inward projection is shaped to allow rim 24 to be passed thereover with a distal movement and to resist passage of rim 24 in a proximal direction, thereby serving to retain tube 13 on shield 11. In the embodiment shown in Fig. 3, projection 26 is located at a distance proximally from interface 23 to capture rim 24 to rest adjacent the interface. Additionally, proximal portion 22 may contain a plurality of sealing rings 27 located proximally from projection 26. Sealing rings 27 are sized to contact cooperatively with an interference fit to a device sized to be placed in proximal portion 22. The cooperative contact substantially serves as a barrier to the passage of microorganisms, allowing the tube to serve as a primary package including the device. In cases where there is no placement of a device in proximal portion 22, rings 27 may be deleted.

Barrier 15 is peeled from surface 18 of flange 14 preferrably by means of a tab 29. Barrier 15 is preferably tamper-indicating and peels without generation of particulates. In the case where the package assembly is not intended as a final package, barrier 15 may be deleted.

Distal portion 21 of tube 13 extends over at least a portion of shield 11. Tube 13 is shown with an internal side 25 of portion 21 preferably having a plurality of inwardly projecting sealing rings 28 which cooperatively contact an external surface 32 of a sidewall 33 of needle shield 11 with an interference. The cooperative contact substantially serves as a barrier to passage of microorganisms. If the intended application of the assembly is not a final sterile package, the rings may be deleted. The extension of distal portion 21 over shield 11 further serves as a secondary protective barrier for substantially reducing the incidence of accidental personnel sticks by a penetration of a sharp medical device such as a needle through sidewall 33 of the shield during improper reshielding or unknowning handling by service personnel during disposal after reshielding.

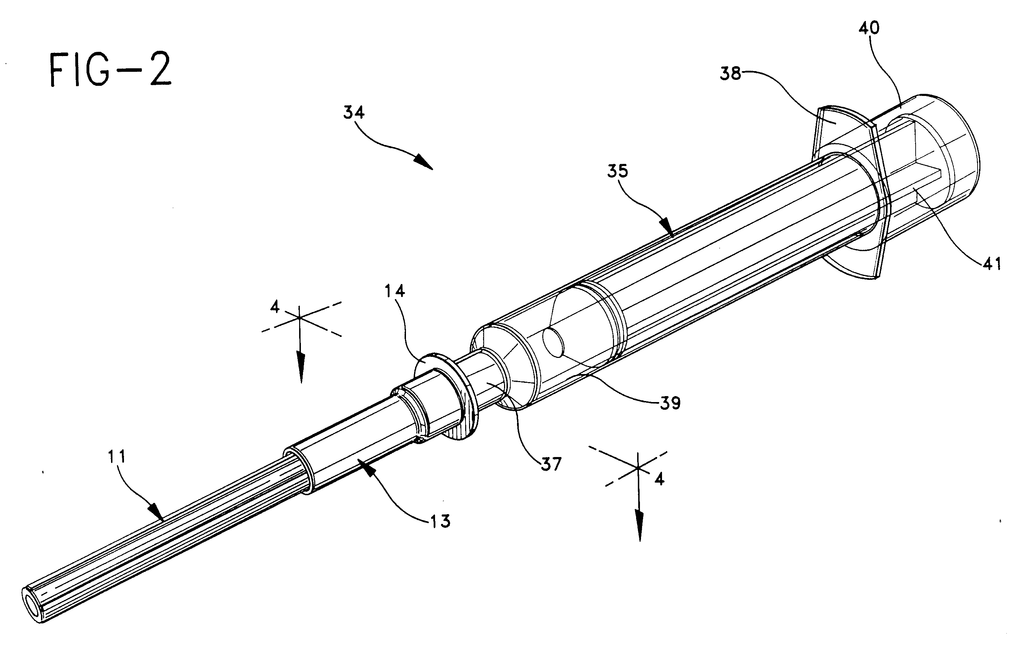

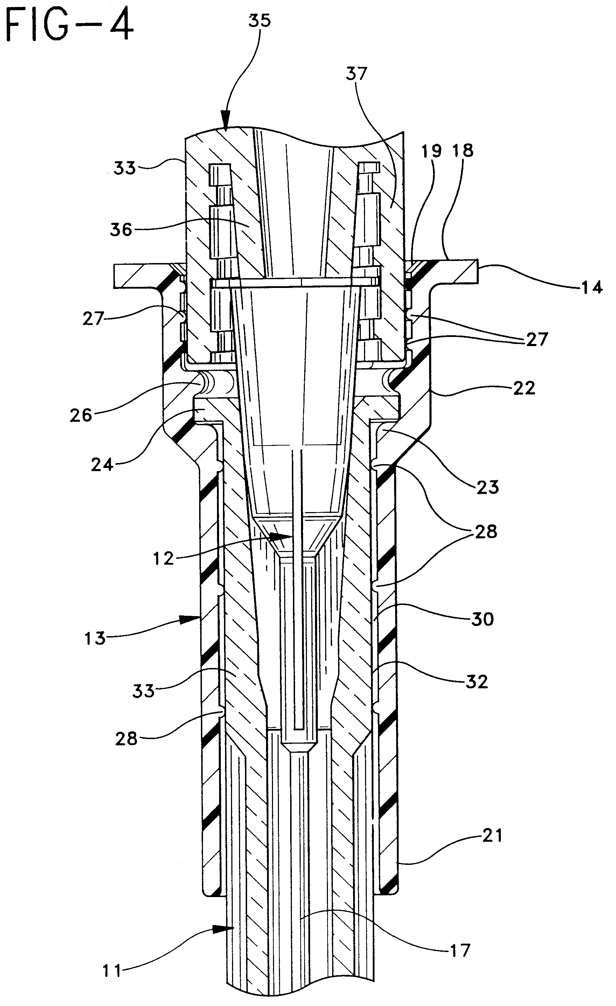

An embodiment of the present invention is shown in Figs. 2 and 4 wherein a packaged syringe assembly 34 includes a syringe 35 with a distal luer tip 36 having an external collar 37. The syringe 35 includes a finger flange 38, a syringe barrel 39, a plunger cap 40 and a plunger assembly 41. Self-contained syringe assembly package 34 further includes hollow tube 13 and needle assembly 12 detachably mounted with interference on distal luer tip 36 with luer hub 16. External collar 37 is fitted into hollow tube 13 having flange 14 and needle shield 11 fitted within it. Syringe assembly 34 includes all other elements of the previously described packaging assembly 10 with the exception of the optional barrier 15. Proximal portion 22 is sized to accept syringe external collar 37 with an interference fit and accept needle shield rim 24. Proximal portion 22 preferably has a plurality of sealing rings 27 located proximally to projection 26 sized to contact cooperatively external collar 37 with an interference fit. The cooperative contact substantially provides a barrier to the passage of microorganisms. The syringe assembly as shown in Figs. 2 and 4 and described above, when terminally sterilized, serves as a self-contained package providing microbial isolation of the fluid path of the syringe and the needle as well as the exterior of the needle, thus eliminating the need for additional packaging.

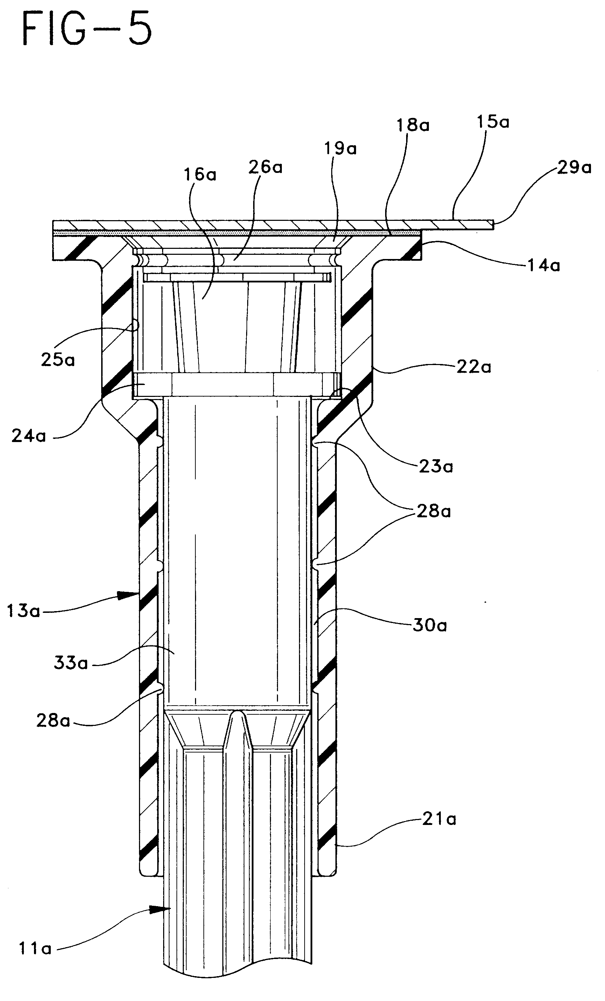

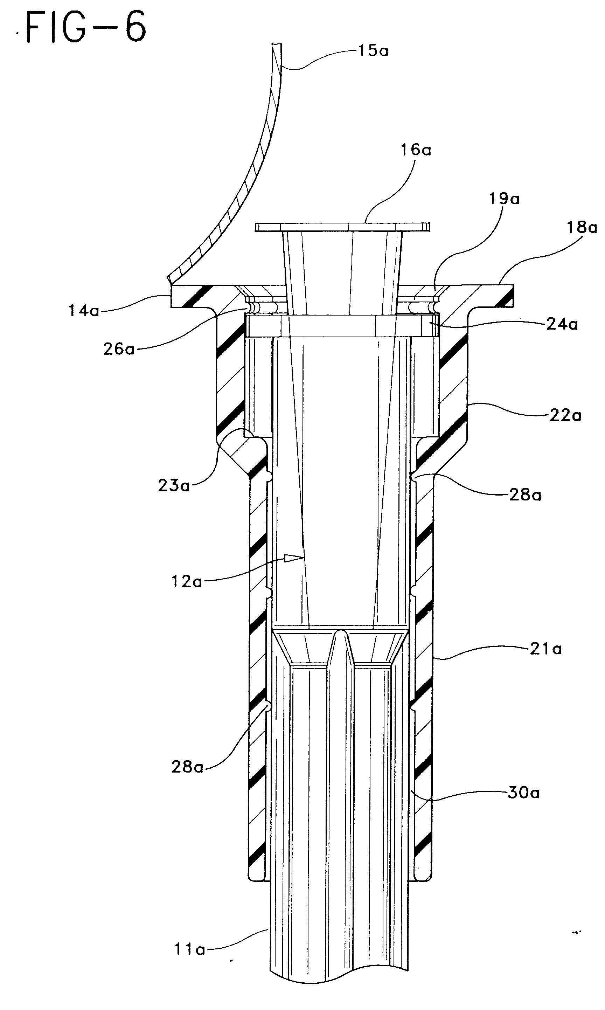

Referring to Figs. 5 and 6, an alternate embodiment of the instant invention is illustrated. In this alternate embodiment the structure of the hollow tube 13 is substantially similar to the embodiment presented in Figs. 1-4. Accordingly, substantially similar components that perform substantially similar functions will be numbered identically to that component of the embodiment of Figs. 1-4 except that a suffix "a" will be used. In this embodiment, an alternate placement is made of projection 26a on proximal portion inner wall 25a. Projection 26a is located proximally to interface 23a. Projection 26a is shaped to allow passage of rim 24a of shield 11a with movement in a distal direction and to resist movement of rim 24a with movement in a proximal direction. The proximal placement of projection 26a on proximal portion inner wall 25a permits proximal movement of shield 11a from interface 23a within portion 22a to allow exposure of needle hub 16a after removal of microbial barrier 15a for mounting a syringe. The microbial barrier 15a can be removed by use of tab 29a, or alternatively, barrier 15a can be provided without a tab. In this case barrier 15a would be removed by a proximal movement of needle shield 11a from shoulder 23a to projection 25a, pushing hub 16a against barrier 15a and detaching it from flange 18a. This ability for proximal movement avoids contact of the sterile hub 16a with the non-sterile package exterior. The design provides for retaining needle shield 11a within hollow tube 13a to provide added needle stick protection after improper disposal.

A benefit of the design of tube 13 is that placement of projection 26 or 26a may be simply accomplished when the tube is formed by injection molding by the use of alternate mold cores. Alternate cores can also be used to size the bore to suit particular size needle shields and for presence or absence and location of sealing rings. Tube 13 may be formed from a plastic, preferably polypropylene, polyamide, polyethylene, polyvinylchloride, polycarbonate and polystyrene, preferably polypropylene, and the like.

The barrier which is used to cover the tube flange can be a molded cap or preferably is a peelable membrane selected from the group consisting of paper, non-woven fabric and film. Medical device packages having all openings with barriers to the passage of microorganisms can be completely assembled and then subjected to terminal sterilization conditions by exposure to ionizing radiation, gas sterilization or any other method for which the materials employed in the manufacture of the device and package components have been qualified. Following this terminal sterilization, the device, or in the case of the self-contained syringe assembly, the fluid path, will remain sterile until the package is opened.

In addition to having the peelable membrane present in several embodiments of the invention as a tamper-evidence, one can employ heat shrink bands or heat stakes at caps or at the several interference junctions present on the device as indicia of package integrity.

In the case of improper disposal, the presence of the non-removable hollow tube over the needle shield substantially serves as a secondary barrier to accidental needle sticks by a needle protruding through the sidewall of the shield. Such a protrusion could occur as a result of improper techniques like reshielding of a needle which was bent during usage. Following this usage, subsequent safe handling by service personnel unknowingly handling the product for disposal is substantially facilitated because the hollow tube will still likely be present on the shield.

Thus it can be seen that the invention of a medical device package having a non-removable secondary barrier which serves as a packaging assembly and a barrier to guard against personnel contact with an improperly disposed of used needle represents an improvement to the state of medical device packaging.

## Claims

1. A packaged syringe assembly (34) comprising:
a syringe (35) having a distal luer tip (36) for detachably mounting a needle and having an external collar (37) on said distal tip;
a needle assembly (12) comprising a proximal luer hub (16) and a needle (17) affixed thereto, said hub (16) detachably mounted on said distal syringe tip (36);
a needle shield (11) having a distal and a proximal end, a cylindrical sidewall having an external surface (32) sized to removably fit at said proximal end with an interference over at least a portion of said proximal luer hub (16) to enclose the needle (17) of said needle assembly (12), said shield (11) having a rim (24) extending outwardly from said external surface (32) at said proximal end thereof;
a formed cylindrical hollow tube (13) having a proximal end and a distal end, and external side and an internal side forming a sidewall, said proximal end and said distal end being open so as to form an open bore (20) extending therethrough, said proximal end of said tube having a flange (14) extending outwardly from said external surface thereof, said flange (14) including a planar surface (18) having an opening (19) to said bore (20), said bore (20) having a distal portion (21) and a proximal portion (22) adjacent to said distal portion at an interface (23), said bore (20) sized so that said distal end of said needle shield (11) fits through said proximal portion (22) and slidably through said distal portion (21) of said bore (20) so that said rim of (24) said shield engages said interface (23); and
said hollow tube distal portion has a length covering at least a portion of said needle shield, said distal portion having within said bore a plurality of sealing rings (28) sized to fit cooperatively with an interference to said external surface (32) of said needle shield (11), said cooperation of said rings and said surface functioning as a barrier to passage of microorganisms and said coverage of said needle shield by said tube thereby serving to prevent accidental personnel sticks by serving as a barrier to penetration of the needle through said sidewall of said shield;
characterized in that said proximal portion (22) of said bore (20) has an inwardly extending projection (26) located proximally to said interface (23), said projection (26) being shaped to allow said rim (24) of said shield (11) to be passed thereover with movement in a distal direction and to resist passage of said rim (24) with movement in a proximal direction;
and in that said proximal portion (22) of said bore (20) has a plurality of raised seal rings (27) located proximally to said inward projection (26) sized to fit cooperatively with said external collar (37) of said syringe luer tip (36) with an interference, said cooperation of said rings and said collar functioning as a barrier to passage of microorganisms.

2. The packaged syringe assembly of claim 1 wherein said inward projection (26) is located at a distance proximally from said interface (23) sufficient to capture said rim (24) of said container (11) adjacent to said interface (23).

3. The packaged syringe assembly of claim 1 wherein said inward projection (26a) is located at a distance proximally from said interface (23a) to permit slidable movement of said rim (24a) between said interface (23a) and said proximal end of said tube (13a).

4. The packaged syringe assembly of claim 1 wherein said flange (14) further includes a barrier (15) to passage of microorganisms.

5. The packaged syringe assembly of Claim 4 wherein said barrier (15) includes a peelable membrane bonded to said flange and selected from the group consisting of paper, non-woven fabric and film.

6. The packaged syringe assembly of claim 1 wherein said tube (13) is formed from a plastic selected from the group consisting of polypropylene, polyamide, polyethylene, polyvinylchloride, polycarbonate and polystyrene.

## Patentansprüche

1. Paketierte Spritzenbaueinheit (34), umfassend:
eine Spritze (35) mit einer distalen Luer-Spitze (36) zum ablösbaren Befestigen einer Nadel und mit einem externen Kragen (37) auf der distalen Spitze;
eine Nadelbaueinheit (12), die eine proximale Luer-Nabe (16) und eine daran befestigte Nadel (17) umfaßt, wobei die Nabe (16) ablösbar auf der distalen Spritzenspitze (36) befestigt ist:
eine Nadelabschirmung (11) mit einem distalen und einem proximalen Ende, einer zylindrischen Seitenwand, welche eine externe Oberfläche (32) besitzt, die zum entfernbaren Befestigen am proximalen Ende mittels Preßpassung über mindestens einen Abschnitt der proximalen Luer-Nabe (16) bemessen ist, um die Nadel (17) der Nadelbaueirheit (12) zu umschließen, wobei die Abschirmung (11) eine Kante (24) aufweist, die sich von der externen Oberfläche (32) am proximalen Ende derselben nach außen erstreckt;
eine profilierte, zylindrische, hohle Röhre (13) mit einem proximalen Ende und einem distalen Ende und mit einer externen Seite und einer internen Seite, die eine Seitenwand bilden, wobei das proximale Ende und das distale Ende offen sind, so daß sie eine offene Bohrung (20) bilden, die sich durch sie hindurch erstreckt, wobei das proximale Ende und die Röhre einen Steg (14) aufweisen, der sich von der externen Oberfläche desselben her nach außen erstreckt, wobei der Steg (14) eine ebene Oberfläche (18) mit einer zu der Bohrung (20) führenden Öffnung (19) umfaßt, wobei die Bohrung (20) einen distalen Abschnitt (21) und einen proximalen Abschnitt (22) angrenzend an den distalen Abschnitt an einem Übergang (23) aufweist, wobei die Bohrung (20) so bemessen ist, daß das distale Ende der Nadelabschirmung (11) durch den proximalen Abschnitt (22) und gleitend durch den distalen Abschnitt (21) der Bohrung (20) hindurchpaßt, so daß die Kante (24) der Abschirmung auf dem Übergang (23) aufsitzt; und
wobei der distale Abschnitt der hohlen Röhre eine Länge besitzt, die mindestens einen Abschnitt der Nadelabschirmung bedeckt, wobei der distale Abschnitt innerhalb der Bohrung eine Anzahl von Dichtungsringen (28) aufweist, die so bemessen sind, daß sie sich zusammenwirkend mit Preßsitz an der externen Oberfläche (32) der Nadelabschirmung (11) anlegen, wobei das Zusammenwirken der Ringe und der Oberfläche als Sperre gegen den Durchtritt von Mikroorganismen wirkt, und die Abdeckung der Nadelabschirmung durch die Röhre dazu dient, unbeabsichtigte Personenstiche zu verhindern, indem sie als eine Sperre gegen das Eindringen der Nadel durch die Seitenwand der Abschirmung dient;
dadurch gekennzeichnet, daß der proximale Abschnitt (22) der Bohrung (20) einen sich nach innen erstreckenden Vorsprung (26) aufweist, der sich proximal zum Übergang (23) hin befindet, wobei der Vorsprung (26) so geformt ist, daß er es der Kante (24) der Abschirmung (11) ermöglicht, durch Bewegung in einer distalen Richtung darübergeschoben zu werden um dem Durchtritt der Kante (24) bei einer Bewegung in proximaler Richtung zu widerstehen;
und daß der proximale Abschnitt (22) der Bohrung (20) eine Anzahl von erhöhten Dichtungsringen (27) aufweist, die proximal zu dem innerseitigen Vorsprung (26) plaziert und so bemessen sind, daß sie zusammenwirkend mit Preßsitz an dem externen Kragen (37) der Luer-Spitze (36) der Spritze ansitzen, wobei das Zusammenwirken der Ringe und des Kragens als Barriere gegen den Durchtritt von Mikroorganismen wirkt.

2. Paketierte Spritzeneinheit nach Anspruch 1 bei der der innerseitige Vorsprung (26) in einem Abstand proximal vom Übergang (23) plaziert ist, der ausreicht, um die Kante (24) des Behälters (11) in der Nähe des Übergangs (23) abzufangen.

3. Paketierte Spritzeneinheit nach Anspruch 1 bei der der innerseitige Vorsprung (26a) in einem Abstand proximal von dem Übergang (23a) plaziert ist, um eine gleitende Bewegung der Kante (24a) zwischen dem Übergang (23a) und dem proximalen Ende der Röhre (13a) zu erlauben.

4. Paketierte Spritzeneinheit nach Anspruch 1, bei der der Steg (14) weiter eine Sperre (15) gegen den Durchtritt von Mikroorganismen umfaßt.

5. Paketierte Spritzeneinheit nach Anspruch 4, bei der die Sperre (15) eine abschälbare Membran umfaßt, die an dem Flansch bondiert ist und aus der Gruppe bestehend aus Papier, Verbundstoffen und Folien gewählt ist.

6. Paketierte Spritzeneirheit nach Anspruch 1, bei der die Röhre (13) aus einem Kunststoff gebildet ist, der aus der Gruppe bestehend aus Polypropylen, Polyamid, Polyethylen. Polyvinylchlorid, Polycarbonat und Polystyren gewählt ist.

## Revendications

1. Assemblage de seringue emballé (34) comprenant:
une seringue (35) comportant une pointe distale luer (36) destinée au montage amovible d'une aiguille et comportant un collier externe (37) sur ladite pointe distale;
un assemblage d'aiguille (12) comprenant un moyeu luer proximal (16) et une aiguille (17) qui y est fixée, ledit moyeu (16) étant monté de façon amovible sur ladite pointe distale de la seringue (36);
un manchon protecteur de l'aiguille (11) comportant une extrémité distale et une extrémité proximale, une paroi latérale cylindrique comportant une surface externe (32), dimensionnée de sorte à être adaptée de façon amovible et par ajustement serré au niveau de ladite extrémité proximale, sur au moins une partie dudit moyeu luer proximal (16). pour renfermer l 'aiguille (17) dudit assemblage d'aiguille (12), ledit manchon protecteur (11) comportant un rebord (24) s'étendant vers l'extérieur à partir de ladite surface externe (32) au niveau de ladite extrémité proximale correspondante;
un tube cylindrique creux formé (13) comportant une extrémité proximale et une extrémité distale, et un côté externe ainsi qu'un côté interne formant une paroi latérale, ladite extrémité proximale et ladite extrémité distale étant ouvertes, de sorte à former un alésage ouvert (20) s'étendant à travers celles-ci, ladite extrémité proximale dudit tube comportant une bride (14), s'étendant vers l'extérieur à partir de ladite surface externe correspondante, ladite bride (14) englobant une surface plane (18) comportant une ouverture (19) vers ledit alésage (20), ledit alésage (20) comportant une partie distale (21) et une partie proximale (22) adjacente à ladite partie distale au niveau d'une surface de jonction (23), ledit alésage (20) étant dimensionné de sorte que ladite extrémité distale dudit manchon protecteur de l'aiguille (11) est insérée à travers ladite partie proximale (22) et par glissement à travers ladite partie distale (21) dudit alésage (20), de sorte que ledit rebord (24) dudit manchon protecteur s'engage dans ladite surface de jonction (23); et
ladite partie distale du tube creux ayant une longueur recouvrant au moins une partie dudit manchon protecteur de l'aiguille, ladite partie distale comportant à l'intérieur dudit alésage plusieurs bagues d' étanchéité (28) dimensionnées de sorte être adaptées par coopération et par ajustement serré sur ladite surface externe (32) dudit manchon protecteur de l'aiguille (11), ladite coopération desdites bagues et ladite surface faisant fonction de barrière au passage de micro-organismes et ladite couverture dudit manchon protecteur de l'aiguille par ledit tube servant ainsi à empêcher des piqûres accidentelles par le personnel en établissant une barrière à la pénétration de l'aiguille à travers ladite paroi latérale dudit manchon protecteur;
caractérisé en ce que ladite partie proximale (22) dudit alésage (20) comporte une saillie s'étendant vers l'intérieur (26), agencée de manière proximale par rapport à ladite surface de jonction (23), ladite saillie (26) étant formée de sorte à permettre le passage dudit rebord (24) dudit manchon protecteur (11) lors d'un déplacement dans une direction distale et à résister au passage dudit rebord (24) en cas d'un déplacement dans une direction proximale;
et en ce que ladite partie proximale (22) dudit alésage (20) comportant plusieurs bagues d'étanchéité surélevées (27), agencées en un point proximal par rapport à ladite saillie interne (26), dimensionnées de sorte à être adaptées par coopération et par ajustement serré sur ledit collier externe (37) de la pointe luer de ladite seringue (36), ladite coopération desdites bagues et dudit collier faisant fonction de barrière au passage de micro-organismes.

2. Assemblage de seringue emballé selon la revendication 1, dans lequel ladite saillie interne (26) est agencée à une distance proximale par rapport à ladite surface de jonction (23), suffisante pour retenir ledit rebord (24) dudit récipient (11) près de ladite surface de jonction (23).

3. Assemblage de seringue emballé selon la revendication 1, dans lequel ladite saillie interne (26a) est agencée à une distance proximale par rapport à ladite surface de jonction (23a) pour permettre un déplacement par glissement dudit rebord (24a) entre ladite surface de jonction (23a) et ladite extrémité proximale dudit tube (13a).

4. Assemblage de seringue emballé selon la revendication 1, dans lequel ladite bride (14) englobe en outre une barrière (15) au passage de micro-organismes.

5. Assemblage de seringue emballé selon la revendication 4, dans lequel ladite barrière (15) englobe une membrane détachable liée à ladite bride et sélectionnée dans le groupe constitué de papier, de tissu non tissé et d'un film.

6. Assemblage de seringue emballé selon la revendication 1, dans lequel ledit tube (13) est composé d'une matière plastique sélectionnée dans le groupe constitué de polypropylène, de polyamide, de polyéthylène, de chlorure de polyvinyle, de polycarbonate et de polystyrène.
